# EUROPEAN PATENT SPECIFICATION

(11) **EP 4 110 453 B1**
(45) Date of publication and mention of the grant of the patent: **30.07.2025**
(21) Application number: 21761383.5
(22) Date of filing: 23.02.2021
(51) Int. Cl.: A61M 39/10, A61M 39/00, F16L 19/02, F16L 47/04, F16L 47/06, F16L 17/00

(54) **CONNECTOR SYSTEM WITH RELEASABLE CONTOUR SEAL FOR A FLUID SYSTEM**
VERBINDUNGSSYSTEM MIT LÖSBARER KONTURDICHTUNG FÜR EIN FLUIDSYSTEM
SYSTÈME DE CONNECTEUR AVEC JOINT DE CONTOUR LIBÉRABLE POUR SYSTÈME DE FLUIDES

(30) Priority: 24.02.2020 US 202062980879 P
(43) Date of publication of application: 04.01.2023
(73) Proprietor: Siemens Healthcare Diagnostics Inc., Tarrytown, NY 10591 (US)
(72) Inventor: KALBFELL, Heiko, 65232 Taunusstein (DE); VIETZE, Andreas, 65812 Bad Soden am Taunus (DE)
(74) Representative: Schweitzer, Klaus
(86) International application number: PCT/US2021/019275
(87) International publication number: WO 2021/173576

(56) References cited:
- EP-A1- 0 151 519
- JP-A- 2015 186 655
- US-A- 4 580 816
- US-A- 4 820 288
- US-A- 5 405 336
- US-A- 6 089 621
- US-A1- 2006 157 975
- US-A1- 2007 216 158
- US-A1- 2010 137 778
- US-B1- 6 345 844
- US-B1- 6 579 717

## Description

### FIELD

Various examples of the invention generally relate to a fitting for a fluid system. Various examples specifically relate to fittings with a releasable contour seal for a fluid system.

### BACKGROUND

In the art, automatic analyzer systems are known which comprise conventional connector systems in which a piercing needle is sealed with an elastic seal. Depending on the liquid used, a different elastic seal is used, which needs to be of a higher quality the more aggressive the liquid. The elastic seal is located on the outer diameter of the piercing needle. The piercing needle is screwed into the interface in order to obtain pressing of the seal. Depending on hardness and material, an appropriate torque is applied.

In other conventional connector systems, e.g. from SMC Corporation, fittings are sealed with an elastic seal from the outside onto the outer diameter of the hose. Colder Products Company (CPC) fittings provide e.g. sealing with an elastic seal in the fitting itself. Fittings by Diba Industries, IDEX Health & Science LLC, or The Lee Company provide e.g. sealing via a flanged hose flat on the interface. These are then pressed from behind by a hollow screw.

EP 0 151 519 A1 disclose s tube coupling having a male body (12), a female body (10) for receiving at least a portion of the male body (12) and at least one peripheral member made from a relatively compressible plastic material molded into a groove within either the relatively rigid male or female body (10, 12). The peripheral member (60, 62) is disposed to become compressed against a surface of the mating body to form a liquid seal. At least two peripheral members (60, 62) disposed in grooves (52, 54) in a tubular shaped end portion of the male body (12) are made of a compressible plastic and serve as liquid sealing and locking means. The female body (10) comprises a receiving end portion (20) with a tapered interior (22) adapted to receive the male tubular end portion (12). As the tubular end portion (50) is inserted into the receiving end portion (20), one of the peripheral members (60, 62) engages the tapered interior surface (22) and becomes compressed there against as the tubular end portion (50) is further inserted. As the tubular end portion (50) is fully inserted a second peripheral member (56) seats itself in a groove (32) in the interior surface of the receiving end portion (20) acting to lock the male (12) and female (10) bodies together. The relatively compressible peripheral members (60, 62) are formed in grooves (52, 54) in the relatively rigid male (12) or female (10) body by insert molding or by simultaneous injection molding of two dissimilar plastics.

JP2015186655A discloses a connector system with a male and a female components, the male comprising at least two ridges and being intended to be fitted in the female component, wherein the ridges are received in annular recesses provided in the inner surface of female component. The female component is made of a material that is softer than the material of the male component so that it deforms as the male component is inserted, which increases the retention force on the male component. The ridges have a triangular shape, wherein the distal end of the ridges being tapered to facilitate the insertion into the female component and into the receiving inner recesses, whereas the coronal end of the ridges is straight and extends perpendicular to the insertion and retraction action of the male component, such that the ridges bite into the inner surface of the female component and thus exert a resisting force when the male and female components are pulled apart, making the disconnection of the connector system more difficult.

When using elastomeric annular parts, such as O-rings, as a seal, then depending on hardness and materials used, an appropriate torque is applied. Further, for generating and maintaining the sealing, conventional connector systems are dependent of the media flowing through, for example the material of an elastomer is chosen to be resistant to the media contained in the fluid system.

### SUMMARY

Therefore, a need exists for improved techniques for connector systems in a fluid system that overcome or mitigate at least some of the mentioned limitations and disadvantages.

This task is solved by the features of the independent claims. Further advantageous examples of the invention are described in the dependent claims.

A connector system, or fitting system, for providing a releasable, fluid-tight seal, in a fluid system is provided.

The present invention is directed to a connector system according to claim 1. The female connector has a cylindrical opening, in other words, a cylindrical bore along a longitudinal axis of the female connector. The cylindrical opening, specifically the longitudinal axis of the cylindrical opening, may be aligned with the longitudinal axis of the connector system. The cylindrical opening may have a length along the longitudinal axis, and an inner diameter perpendicular to the longitudinal axis, i.e. in the radial direction with regard to the longitudinal axis. The inner diameter may be the same inner diameter over the length of the cylindrical opening, specifically over a length, in which sealing protrusions of a male connector of the connector system may come into contact with the inner circumferential surface of the cylindrical opening.

The connector system further comprises a male connector aligned with the longitudinal axis, which has a tubular end section along the longitudinal axis.

The tubular end section defines a cylindrical inner fluid passage along the longitudinal axis through the tubular end section and the male connector. In the tubular end section, a circumferential wall may be formed by an inner circumferential surface of the tubular end section, and an outer circumferential surface of the tubular end section. The fluid passage is defined by the inner circumferential surface of the tubular end section, wherein the outer circumferential surface of the tubular end section defines features for connecting or coupling the male connector to the female connector.

The male connector, specifically the tubular end section of the male connector, is inserted in an insertion direction along the longitudinal axis into the cylindrical opening of the female connector, wherein a fluid flow is possible through the connector system through the fluid passages of the male and the female connectors.

A mechanical coupling between the male and the female connectors, and specifically a sealing of the fluid system to the outside, may be effected between the tubular end section of the male connector and the cylindrical opening of the female connector, specifically the outer circumferential surface of the tubular end section of the male connector and the inner circumferential surface of the cylindrical opening of the female connector.

By the outer circumferential surface of the tubular end section, annular protrusions are formed.

The annular protrusions are integrally formed in the male connector, in particular in the tubular end section. In other words, the annular protrusions are made of the same material as the male connector and are produced in the same production steps monolithically as part of the male connector.

The annular protrusions extend extend in the radial direction over the outer circumferential surface with regard to the longitudinal axis.

The annular protrusions are inserted together with the tubular end section into the cylindrical opening when connecting the male and the female connectors. In such a way, the tubular end section is characterized by a sealing contour defined by the annular protrusions, which may be an undulating contour.

The tubular end section has an annular intermediate protrusion, which has an outer diameter bigger than an inner diameter of the cylindrical opening.

The outer diameter of the intermediate protrusion is slightly bigger than the inner diameter of the cylindrical opening, so that the inner circumferential surface of the cylindrical opening is slightly deformed, when the intermediate protrusion is inserted with the tubular end section into the cylindrical opening, in other words a press-fit is formed between the male and the female connectors. The ratio between the inner and outer diameters may be determined based on design rules for press fits, and specifically based on the materials used for the male and female connectors and the fluid media and pressure in the fluid system.

The tubular end section further has an annular rear protrusion, which is located adjacent to the intermediate protrusion along the longitudinal axis. When a direction along the longitudinal axis, in which the male connector is inserted into the female connector, is referred to as insertion direction, then the rear protrusion is located behind the intermediate protrusion with regard to the insertion direction. In other words, when inserting the tubular end section, the rear protrusion is inserted after the intermediate protrusion.

The rear protrusion has an outer diameter, which is bigger than the outer diameter of the intermediate protrusion. In this way, between the inner circumferential surface of the cylindrical opening and the rear protrusion, a press fit is formed, the elastic deformation of the inner circumferential surface of the cylindrical opening is bigger than for the intermediate protrusion.

When the tubular end section, together with the annular protrusions, is inserted into the cylindrical opening, the intermediate protrusion and the rear protrusion each form a press-fit with the inner circumferential surface of the cylindrical opening, wherein a fluid-tight seal between the male connector and the female connector is effected. In such a way the insertion force needed to insert the male connector into the female connector may be increased from the intermediate protrusion to the rear protrusion.

Thereby, a connector system for leakage-free sealing with releasable connections (e.g. for a piercing needle or a fitting) in fluid systems is provided.

The disclosed techniques do not require any elastomeric shaped part (O-ring, etc.) for generating and maintaining the sealing. The device is independent of and resistant to the media flowing through, wherein the fluid material and a material of an elastomer are to be chosen to be compatible with each other.

The tubular end section further comprises an annular front protrusion, which is located in front of the intermediate protrusion with respect to the insertion direction, and which has an outer diameter, which is the same or smaller, specifically the same, as the inner diameter of the cylindrical opening. In such a way, the front protrusion may be inserted before the intermediate and rear protrusions, when inserting the tubular end section into the cylindrical opening, wherein the front protrusion may not effect a substantial frictional force and/or a press fit to the cylindrical opening, and may be used as a guide for centering and inserting the male connector.

The outer diameters of the protrusions are defined by the outermost points in radial direction on the circumferential outer surfaces of each protrusion. The protrusions have a convex shape in the region, where they make contact with the circumferential surface of the cylindrical opening. Such convex regions have vertices, which are in contact with the cylindrical opening, in other words, the vertices of the protrusion contours in radial direction define the outer diameters of the protrusions. In other words, a protrusion has an outer radius, which may be a circle with the outer diameter as a radius.

In various examples, the circles defined by the outer diameters, and/or the outer radii, i.e. one circle for each protrusion, may define a conical surface, or a conical shape, specifically a truncated cone, around the longitudinal axis. A truncated cone may be defined by the outer radiuses of all protrusions, in other words, the vertices, i.e. the outer radii, of the protrusions lie on a virtual lateral surface of a truncated cone. The conical surface may be a rotationally symmetrical surface with a constant inclination angle and may be aligned with its axis along the longitudinal axis.

In such a way, the lateral i.e. circumferential surface, may define an angle with the longitudinal axis, in other words, the conical surveys may have an inclination angle relative to the longitudinal axis, which may be in the range of 0.1 to 2 degree, specifically 0.5 to 1 degree. This inclination angle may be used to define the insertion and sealing characteristics of the connector system.

A contour of a protrusion is defined by its outer surface along the longitudinal axis. In other words, when a section, or cross section, is created between the outer circumferential surface of a protrusion and a plane including the longitudinal axis, this cross-section defines an outer contour of a protrusion.

The contour of a protrusion has a region, which is in contact with the cylindrical opening in a press-fit. For example, in a press fits the circumferential wall of the cylindrical opening is slightly deformed, such that a sealing region, i.e. sealing surface, around and/or including the vertex of a protrusion is in contact with the surface of the cylindrical opening. In other words, during a press-fit, when a seal is effected and maintained, regions on both sides of an outer radius of a protrusion may be in contact with the surface of the cylindrical opening.

The region of the contour of a protrusion, or of all protrusions, which is in contact with the female connector in a press-fit, has the same convex shape around its vertex in both insertion direction and the opposite extraction direction along the longitudinal axis. In other words, the contour of the protrusion along the longitudinal axis is symmetrical with regard to its vertex, i.e. the contour is symmetrical with regard to a symmetry axis perpendicular to and through its vertex.

A symmetrically round shape effects that an insertion force in insertion direction, i.e. a force needed to insert the male connector and/or to move the male connector in insertion direction, when the seal may be already effected, and extraction force, opposite to the insertion direction, i.e. a force needed to extract or remove the male connector from the female connector, may be the same. The connector system according to the present disclosure may be thereby a releasable connector system.

The protrusions may be rotationally symmetrical with regard to the longitudinal axis.

The protrusions, in particular the intermediate and rear protrusions, may have the same contour arranged in different radial distances from the longitudinal axis.

The protrusions may be arranged directly adjacent to each other, wherein they may be connected by concave regions in the contour. The two concave regions connecting the three convex protrusions, may have the same contour, i.e. they may be circular contours with the same radius, and/or with different center positions.

The protrusions may have a circular contour with the same radius, specifically the vertex may be comprised in a circular convex shape.

The convex contours may be defined as circular contours by respective center positions and radii, in a cross-sectional plane including the longitudinal axis.

The protrusions, in particular the intermediate and the rear protrusions, may have the same contour with the same radii, wherein the center positions of the protrusions may be arranged in different distances from the longitudinal axis, and/or in different positions along the longitudinal axis. In such a way, the convex outer contours of the protrusions define the inclination angle of the conical surface.

In various examples, the differences between the protrusion outer diameters may be multiples of a radial step size. In other words, the difference between the front protrusion and the intermediate protrusion, and the difference between the intermediate protrusion and the rear protrusion may be the same, specifically the distances of the circular centers along the longitudinal axis, and the distances of the circular centers and/or the outer radii/diameters and/or vertices in radial direction.

The female connector may further form an inner fluid passage along the longitudinal axis, adjacent to the cylindrical opening, wherein the inner fluid passages of the female and male connectors have the same cross section, specifically diameter.

The tubular end section of the male connector may form an end surface, which may be a front face surface of the tubular end section in insertion direction. When fully inserted in the cylindrical opening, the end surface of the tubular end section may abut an end surface of the fluid passage of the female connector in longitudinal direction. The end surface of the fluids passage of the female connector may be a surface connecting the cylindrical opening and the inner fluid passage. In such a way, the end surfaces of the male and the female connector may be inclined surfaces, specifically perpendicular surfaces with regard to the longitudinal axis.

Thereby, when fully inserted, the inner fluid passage of the male and the female connectors may be seamlessly merged into each other, in other words may blend into each other without step and/or undercut, they may form a continuous inner surface through the connector system.

It is to be understood, that end surfaces providing a continuous inner fluid passage may not be necessary in all examples, wherein, in various examples, no stopping surfaces or mechanisms may be present within the cylindrical opening.

The connector system may further comprise a union nut, or swivel nut, for inserting and/or holding the male and female connectors together.

One of the female or the male connector may further comprise a union thread on its outer circumferential surface, wherein the other one of the female or the male connector may comprise an annular union protrusion on its outer circumferential surface. A union nut may be arranged around the male and the female connector in an inserted state, specifically may extend around the union thread and the union protrusion, and the interface between those. The union nut may further comprise an inner thread engaging the union thread, and may mechanically rotatably engage the union protrusion, such that, when the union nut may be rotated with regard to the union thread, the union nut may be shifted along the longitudinal axis and, by mechanical engagement with the union protrusion may hold and/or shift the union protrusion. In such a way, the female and male connectors may be held together, wherein they may be further inserted, in other words, the seal may be tightened, or released, by the union nut.

The male connector is made of, i.e. consist of, a single material. The female connector is made of a single material. The male and the female connector are made of the same single material.

The connector system does not comprise an elastomeric material, except the monolithic male and female connectors. Specifically, no additional annular elastomer, for example, an O-ring may be comprised in the connector system for generating the seal.

The material of the female connector is softer, i.e. have a bigger elasticity, than the material of the male connector.

The material of the female connector may have a young modulus in the range of 1000-5000 MPa, specifically 2500-4000 MPa.

The material of the male connector may have a young modulus in the rage of 2000-4000 MPa, specifically 3000-4000 MPa.

In various examples, the materials of the male and the female connector have the same material composition, however, they may have different composition component ratios, wherein they have different elasticities, i.e. young moduli.

The young modulus of the single material of the male connector may not deviate by more than 50%, or specifically 30%, or more specifically 20%, from the young modulus of the single material of the female connector.

In other words, the material of female connector has a bigger elastic deformation, i.e. in the elastic deformation range, as opposed to the plastic deformation range, than the material of the male connector.

In various examples, the material of the female connector may not be an Elastomer, but e.g. thermoplastic material, or a Duroplast or Duromer, which may allow a small elastic deformation for forming a press fit, i.e. interference fit, with the annular protrusions of the male connector.

In various examples, a press-fit, or interference fit may be defined as follows. The female connector may have a constant inner diameter D, or height, as the opening may in some examples not be cylindrical. The male connector has an outer diameter at the protrusions (or height, if in some examples the protrusions are not rotationally symmetrical. The opening in the female connector is configured to receive and to provide an interference fit or press fit to the male connector, specifically to the sealing protrusions. The term interference fit shall be interpreted broadly as including the joining of any two mating parts such that one or the other (or both) parts slightly deviate in size from their nominal dimension, thereby deforming each part slightly, each being compressed, the interface between two parts creating a union of extremely high friction. In various examples, the materials of the female and the male connector may be chosen, so that substantially only the female connector is deformed. The word interference refers to the fact that one part slightly interferes with the space that the other is occupying in its nominal dimension. Optionally, the interference fit can be configured to require at least a predefined force, e.g. a force having a specific value in the range of 10-800 N, to remove the male connector from the female connector.

In various examples, the female inner diameter may be constant, or it can be sloped or have steps through the inner length of the female connector up until a shoulder that prevents the male connector, e.g. end face of male connector, from advancing any further into the female connector. Thus, the female inner diameter may be constant along the longitudinal axis, thus it will be understood that the female member provides the ability to adjust the distance between the male and female connectors.

In various examples, the material characteristics may be chosen vice versa, such that the male connector may be made of the softer material.

In general, the male connector and/or the female connector may be monolithic, plastic injection-molded parts. The male connector, specifically may be made of a Polyetheretherketon (PEEK) material.

The female and male connectors may be configured to be connected in a linear movement, without rotational movement, along the longitudinal axis, to generate the fluid-tight seal. In various examples the front protrusion may be used as a centering guide in the cylindrical opening during connecting. Specifically, the connectors may be moved linearly along the longitudinal axis relative to each other while the fluid-tight seal is maintained. Allowing for linear movement, while the fluid-tight seal may be maintained, over a predefined distance, gives the connector system mechanical stability and tolerance with respect to mechanical stress on the connector system from outside.

In various examples, between the intermediate protrusion, the rear protrusion, and the inner circumferential surface of the cylindrical opening, a closed labyrinth may be generated, in other words, a labyrinth seal may be provided.

In various examples, the male connector may comprise exactly three annular protrusions, i.e. sealing rings. The sealing rings may be arranged from an end face, which is to be inserted into the female connector first of the tubular end section, in an ascending order according to their outer diameters, such as to form an undulating ascending sealing contour. Using the same round contours for all convex and concave sections in the undulating contour provide improved sealing characteristics. Specifically, using circular contours with the same radius for all convex contours and another same radius for all concave sections between the convex sections of the sealing contour, further improves the sealing characteristic.

A coupling system for a hose is disclosed herein (not part of the invention). The coupling system comprises a connector, which has a cylindrical wall defining an inner fluid passage along a longitudinal axis. The connector further comprises an outer circumferential surface defining an outer thread and a conical section, which is integrally formed in the connector, specifically is integrally formed in the cylindrical wall of the connector, wherein the diameter of the conical section is reduced towards an end of the connector in insertion direction into a hose. In other words, the outer circumferential surface at an end of the connector, which is to be inserted into an end of a hose, comprises a conical surface, whose diameter is inclining or reduced or tempered towards the end of the connector along the longitudinal axis. The conical section, or the conical surface may be rotationally symmetrical with regard to the longitudinal axis.

In various examples, the conical surface is integrally formed within the cylindrical wall of the connector, specifically from a front end face of the connector. For example, the conical section may have a perpendicular end surface on a Beck and with regard to the insertion direction into the hose.

In various examples, the connector may have the inner fluid passage defined as a continuous fluid passage through the connector and specifically through the integrally formed conical section. The conical section may be integrally formed of the same material with the connector as a plastic injection molded part.

The coupling system may further comprise a tubular clamping nut, which is arranged around the connector along the longitudinal axis. The tubular clamping nut may function as a swivel or union nut.

The tubular clamping nut may have an inner circumferential surface defining an inner thread engaging the outer thread of the connector, wherein rotation of the threads relative to each other, causes the clamp to move relative to the connector along the longitudinal axis.

The inner circumferential surface of the tubular clamping nut further defines a clamping surface around the conical surface of the connector.

By rotation of the clamp around the connector a clamping distance between the clamping surface and the integrally formed conical surface is reduced, in order to press a hose wall of the hose, in which the connector has been inserted, against the conical surface. Thereby, a mechanical coupling and a liquid-tight seal between the hose and the connector may be effected.

The clamping surface may extend rotationally symmetrically around the conical surface, wherein, during rotation of the clamp, the conical and the clamping surfaces are shifted along the longitudinal axis towards each other.

In various examples, the clamping surface and the conical surface may be parallel surfaces, and during rotation of the clamp, they are shifted in parallel to each other, such that a direct, or perpendicular, distance between the surfaces is the same at all points along the conical surface.

It is to be understood, that the surfaces can also be angled with regard to each other.

The coupling system may be provided at an end of the male connector and/or the female connector of the connector system according to the present disclosure.

The present invention further provides an automated analysis machine comprising at least one connector system according to claim 1.

In a preferred embodiment, the automated analysis machine comprises one receiving positions for a liquid vessel, or a plurality of receiving positions for a liquid vessel.

In a preferred embodiment, the automated analysis machine comprises one robotically displaceable transfer arm or a plurality of robotically displaceable transfer arms.

The present invention further discloses the use of least one connector system and/or a coupling system according to the invention in an automated analysis machine.

The term "biofluid" refers to a human or animal body fluid, which may be, but is not limited to, blood.

Although specific features described in the above summary and the following detailed description are described in connection with specific examples, it is to be understood that the features may not only be used in the respective combinations, but may also be used isolated, and features from different examples may be combined with each other, and correlate to each other, unless specifically stated otherwise.

Therefore, the above summary is merely intended to give a short overview over some features of some embodiments and implementations and is not to be construed as limiting. Other embodiments may comprise other features than the ones explained above.

### BRIEF DESCRIPTION OF THE DRAWINGS

In the following, concepts in accordance with exemplary embodiments of the invention will be explained in more detail with reference to the following drawings, in which like reference numerals refer to like elements.
Fig. 1 schematically illustrates a connector system in a liquid system, according to embodiments of the invention.
Fig. 2 schematically illustrates the piercing needle inserted into the manifold of Fig. 1, according to embodiments of the invention.
Fig. 3 schematically illustrates the piercing needle of Figs. 1 and 2, according to embodiments of the invention.
Fig. 4 schematically illustrates a connector system, according to embodiments of the invention.
Fig. 5 schematically illustrates the connector system of Fig. 4 with two tubular clamping nuts, according to embodiments of the invention.
Fig. 6 schematically illustrates a cross-section of a connector system, according to embodiments of the invention.
Fig. 7 schematically illustrates the connector system of Fig. 6 with a union nut.
Fig. 8 schematically illustrates a coupling system with a hose, not according to embodiments of the invention.
Fig. 9 schematically illustrates a tubular end section of a male connector, according to embodiments of the invention.
Fig. 10 schematically illustrates an inclination angle of protrusions, according to embodiments of the invention.
Fig. 11 schematically illustrates the tubular end section of Fig. 9 inserted into a cylindrical opening of a female connector.

### DETAILED DESCRIPTION

The above and other elements, features, steps, and concepts of the present disclosure will be more apparent from the following detailed description in accordance with exemplary embodiments of the invention, which will be explained with reference to the accompanying drawings.

The drawings are to be regarded as being schematic representations, and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling.

In conventional connector systems, a piercing needle is sealed with an elastic seal. Depending on the fluid used, a different elastic seal is used, which needs to be of a higher quality the more aggressive the fluid. The elastic seal is located on the outer diameter of the piercing needle. The piercing needle is screwed into the interface in order to obtain pressing of the seal. Depending on hardness and material, an appropriate torque is applied.

In other conventional techniques, SMC fittings are sealed with an elastic seal from the outside onto the outer diameter of the hose. CPC fittings provide sealing with an elastic seal in the fitting itself. Fittings by Diba Industries, IDEX Health & Science LLC, or The Lee Company provide e.g. sealing via a flanged hose flat on the interface. These are then pressed from behind by a hollow screw. Depending on hardness and material, an appropriate torque is applied.

Using elastomeric annular parts, such as O-rings etc., for generating and maintaining the sealing, conventional connector systems are dependent of the media flowing through, wherein the material of an elastomer is to be chosen to be resistant to the liquid media.

In the following, various mechanisms with respect to leakage-free sealing with releasable connections (e.g. for a piercing needle or a fitting) in fluid systems will be described.

The disclosed techniques do not require any elastomeric shaped part (O-ring, etc.) for generating and maintaining the sealing. The device is independent of and resistant to the media flowing through, wherein the fluid material and a material of an elastomer are not to be chosen to be compatible with each other.

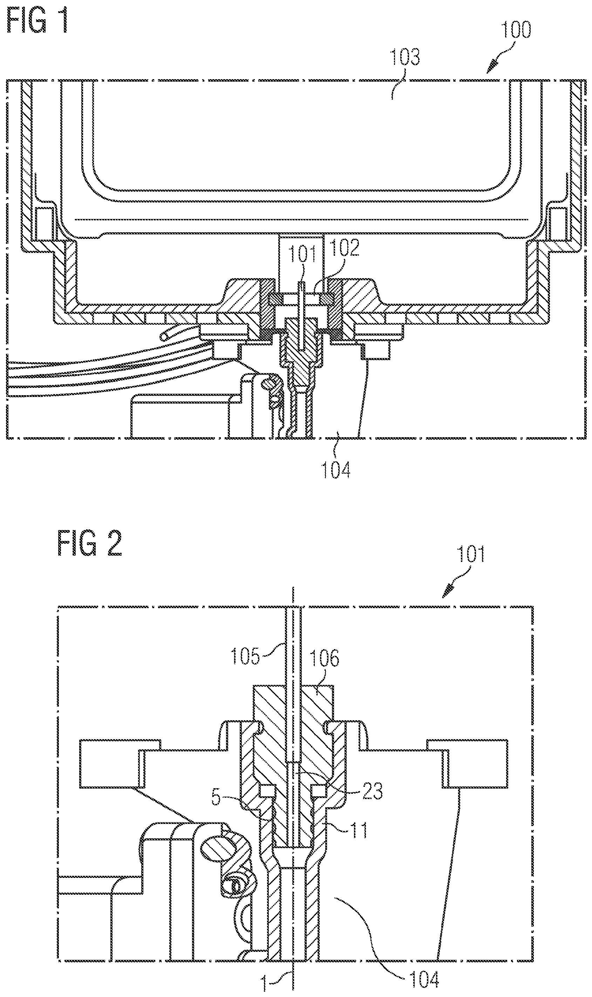

Fig. 1 schematically illustrates a connector system 100 in a liquid system, according to embodiments of the invention.

As can be seen in Fig. 1, a piercing needle 101 is used to penetrate a septum 102 on a storage bottle 103. The piercing needle 101 is placed in a manifold 104. Through the piercing needle 101, the cleaning liquid from the storage bottle 103 is introduced via the manifold 104 into the liquid system of an analyzer.

In the example of Fig. 1, the piercing needle 101 may correspond to a male connector, wherein the manifold 104 may correspond to a female connector. A liquid tight seal is provided between the piercing needle 101 and the manifold 104.

Fig. 2 schematically illustrates the piercing needle 101 inserted into the manifold of Fig. 1 in more detail, according to embodiments of the invention.

As can be seen in Fig. 2, the piercing needle 101 is composed of the hollow needle 105 and a fitting 106, which may correspond to a male connector 20 according to the present disclosure. The hollow needle 105 is pressed in the fitting 106. The fitting 106 has an inner fluid passage 23 defined along a longitudinal axis 1.

The manifold 104 has a cylindrical opening 11, wherein a tubular end section with a sealing contour 5 of the fitting 106 is inserted.

As can be further seen in Fig. 2, the manifold comprises a threaded cylindrical opening with a diameter bigger than the cylindrical opening 11 and directly adjacent to the cylindrical opening 11, in which a threaded section of the fitting 116 is situated in threaded engagement with the manifold 104. The fitting 106 is screwed into the manifold 104 and held in place by the threads.

The sealing contour 5 of the fitting 106 is located in the outer circumferential surface of the fitting 106 inserted into the cylindrical opening 11 and a press-fit providing a liquid-tight sealing.

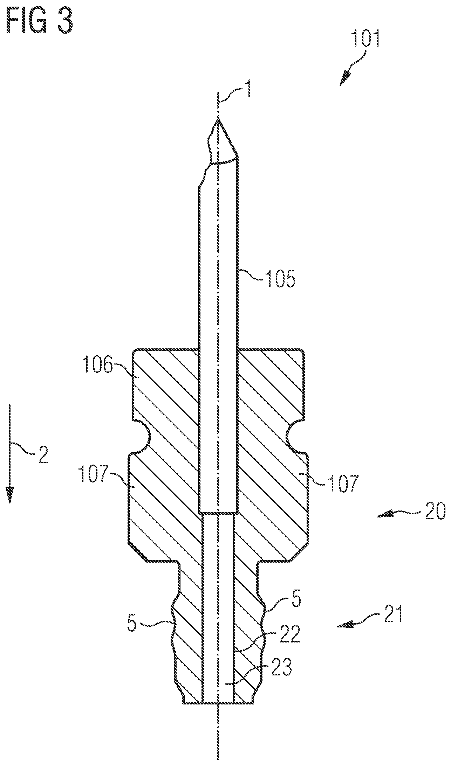

Fig. 3 schematically illustrates the piercing needle 101 of Figs. 1 and 2, according to embodiments of the invention.

As depicted in Fig. 3, the piercing needle 101 comprises the hollow needle 105, which is pressed into a fitting part 106.

The fitting 106 corresponds to a male connector 20 according to the present disclosure. The fitting 106 comprises a tubular end section 21 with a sealing contour 5, and a threaded section with an outer thread 107. The outer thread 107 may be used to screw the piercing needle into a manifold, which may correspond to a female connector according to the present disclosure.

Inside the fitting 106, the hollow needle 105 and a fluid passage 23 are aligned with the longitudinal axis 1. The fluid passage 23 is formed by an inner circumferential surface 22 of the tubular end section 21.

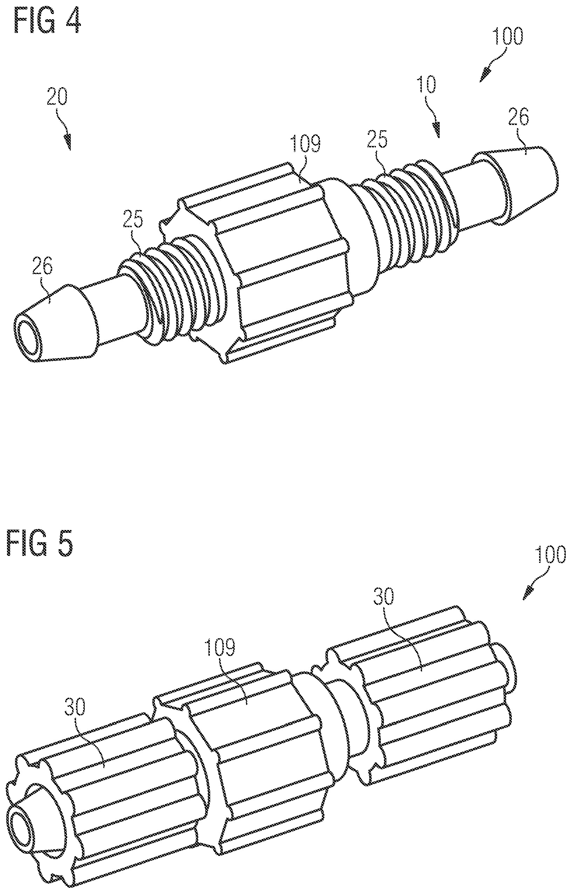

Fig. 4 schematically illustrates a connector system 100, according to embodiments of the invention.

The connector system 100 comprises a female connector 10 and a male connector 20, which are held together by a union nut 109. On an outside end, which is the other end of each of the female and male connectors along the longitudinal axis from the end, where the liquid-tight seal is generated, a coupling system for coupling a hose to each of the female and male connectors, according to the present disclosure is defined.

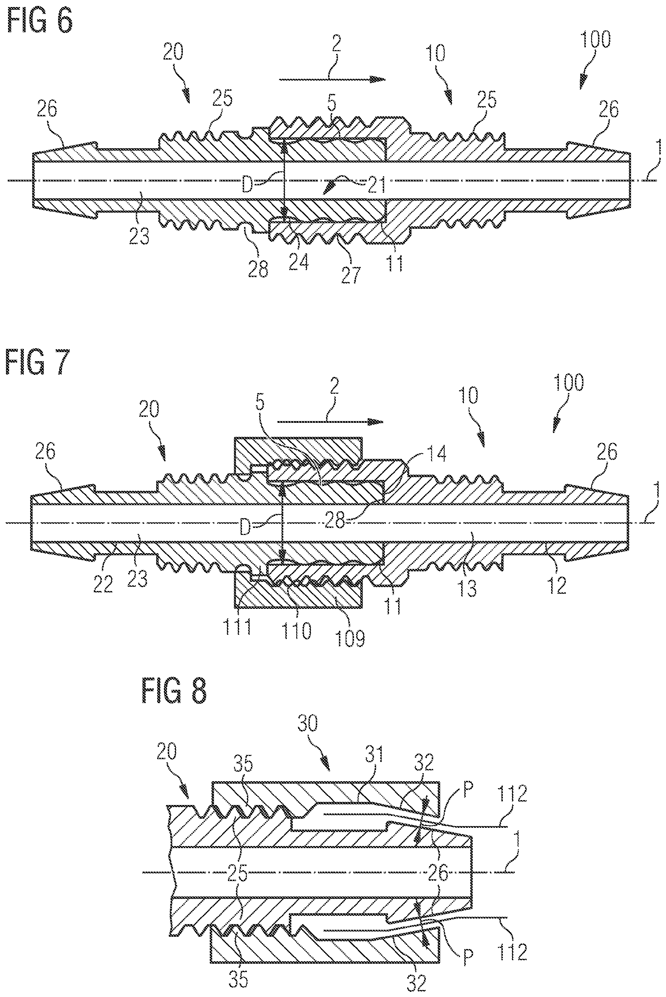

For coupling system into a hose (not part of the invention), an outer thread 25 and a conical section 26 are formed on each of the outer ends, which will be described in more detail with reference to Fig. 8.

Fig. 5 schematically illustrates the connector system of Fig. 4 with two tubular clamping nuts 30, according to embodiments of the invention. The tubular clamping nuts 30 will be described in more detail with reference to Fig. 8.

As can be seen in Figs. 4 and 5, the fitting pair, i.e. the connector system, is composed of a fitting with the sealing contour 5 and of a fitting with the cylindrical bore 11. To produce a connection between both of them, they are screwed together with a union nut 109.

Fig. 6 schematically illustrates a cross-section of a connector system 100, according to embodiments of the invention.

The connector system 100 comprises a male connector 20, which is pressed into a female connector 10 in insertion direction 2.

Each of the male connector 20 and the female connector 10 are aligned along the longitudinal axis 1 and may further have a coupling end to be coupled to a hose.

Each of the coupling ends define a conical section 26 to be inserted into a hose and an outer thread 25 on which a tubular clamping nut for clamping the hose may be arranged.

The male connector 20 further comprises a tubular end section 21, which is inserted into a cylindrical opening 11 of the female connector 10 having an inner diameter D.

The tubular end section 21 has an outer circumferential surface 24, on which a sealing contour 5 is formed. In an inserted state, sealing contour 5 defines 3 annular protrusions in press-fit contact with the inner circumferential surface of the cylindrical opening 11.

On an outside surface of the female connector 10, in a radial direction over the tubular end section 21, the female connector 10 has an outer thread 27 defined, which is a thread for engagement with a union nut to hold the female and male connectors together.

Fig. 7 schematically illustrates the connector system of Fig. 6 with a union nut 109.

As can be seen in Fig. 7, the union nut 109 causes a linear pressing of the male connector 20 with the sealing contour 5 and of the female connector 10 with the cylindrical bore 11, without the female and male connectors having to be rotated.

Union nut 109 comprises an inner thread 110 in engagement with the outer thread 27 of the female connector 10. The union nut one hundred and 9 further engages in union protrusion 111, in order to hold the male connector 20 in place.

As depicted in Fig. 7, a continuous inner fluid passage is formed by the fluid passage 23 of the male connector 20 and the fluid passage 13 of the female connector 10. At the end of the cylindrical opening 11, the fluid passage 23 of the female connector having a smaller diameter is joined to the cylindrical opening 11 in a step, or in other words by shoulder or end surface 14 of the female connector 10. Onto this end surface 14, an end surface 28 of the tubular end section 21, which is the end face of the tubular end section 21 in insertion direction 2, abuts, wherein the in the fluid passages are seamlessly merged, without forming an undercut or a groove. In this regard, the front protrusion may mechanically support the tubular wall section, if it has the same outer diameter as the inner diameter of the cylindrical opening 11.

Fig. 8 schematically illustrates a coupling system with a hose, not according to embodiments of the invention.

As illustrated in Fig. 8 (not according to the invention), a hose 112 may be engaged elastically over a conical section 26, which is a one-step conical shoulder, of a female or male connector until it reaches a stop surface, i.e. is fully inserted. A release of the hose 112 is prevented with a tubular clamping nut 30.

A conical section is integrally formed in a cylindrical wall of a connector 20. In particular, the conical section 26 comprises a conical lateral surface, which is angled towards the end face of the connector 20 and in an arrow or hook shape, and which is inserted into the hose 112.

The tubular clamping nut 30 comprises on an inner circumferential surface 31 an inner thread 35 engaging the outer thread 25 of the connector. The inner circumferential surface 31 further forms a clamping surface 32, which extends around the lateral surface of the integrally formed conical section 26.

When the clamping nut 30 is rotated around the connector 20, it is linearly shifted along the longitudinal axis 1, whereby the distance P between the lateral surface of the conical section 26 and the clamping surface 32 is reduced. In this example, the lateral surface of the conical section 26 and the clamping surface 32 are parallel surfaces, which are shifted in parallel with regard to each other, when the clamping nut 30 is rotated. Thereby, the hose 112 may be fixed between the one-step conical shoulder and the clamping nut 30, wherein the hose wall may be pressed against the conical section 26 without being shifted further along the conical section 26.

In particular, this coupling system may be comprised of the female 10 and/or the male connector 20, according to the present disclosure, on to a plane and side, which is situated opposite of the tubular end section 21 and/or the cylindrical opening 11.

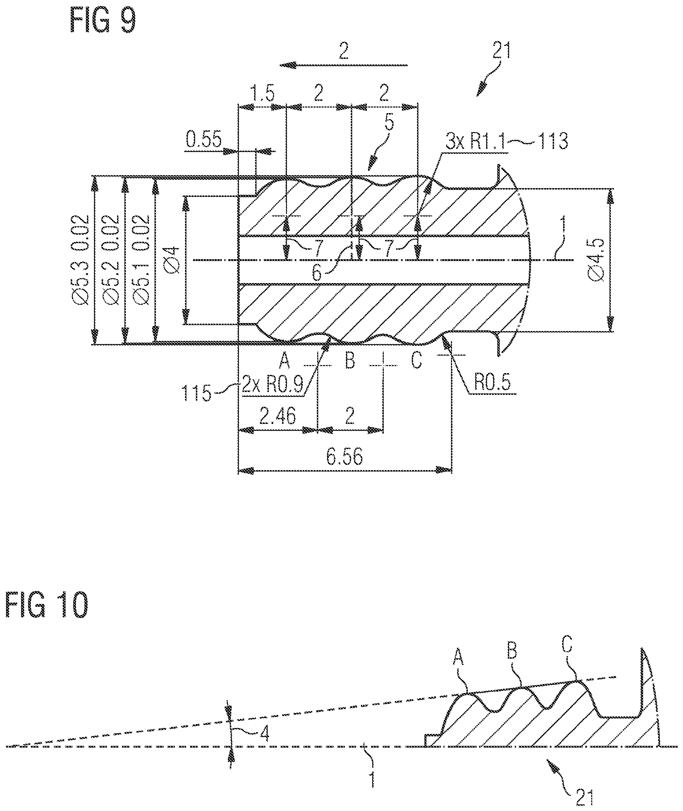

Fig. 9 schematically illustrates a tubular end section 21 of a male connector 20, according to embodiments of the invention.

As can be seen in Fig. 9, the sealing contour 5 consists of three protrusions A, B, C, which may be referred to as annular sealing protrusions, and which are circumferential rings extending over a circumferential base surface of the tubular end section 21, around the longitudinal axis, and integrally formed within the tubular section 21 of the same material as the whole male connector 20.

The front protrusion A extends over an outer diameter of 4 mm of the end face of the tubular end section 21 and is located on a front side with respect to insertion direction 2.

The intermediate protrusion B extends in a radial direction over the front protrusion A, and is situated directly adjacent to front protrusion A, wherein in the contour 5, the convex contours 113 of protrusions A and B are connected by a concave contour region 115.

The intermediate protrusion C extends in a radial direction over the intermediate protrusion B, and is situated directly adjacent to intermediate protrusion B, wherein in the contour 5, the convex contours 113 of protrusions B and C are connected by a concave contour region 115, which is the same as between protrusions A and B.

These protrusions all have the same profile, i.e. the same convex profile 113 and the same concave profile 115, and also the same distance between the rings A, B and rings B, C. However, the different sealing protrusions are arranged in different radial distances from the longitudinal axis, such that a force for introducing the tubular end section 21 into the cylindrical opening 11 is regularly increased along the longitudinal axis 1. In other words, there may be no other convex sections in the sealing profile between the convex sections of the sealing protrusions A, B, C.

The ring A with an outer diameter Ra of 5.1 mm serves for centering in the cylindrical bore 11 of the female connector 10. The cylindrical bore 11 has an inner diameter D of 5.1 mm.

The ring B has an outer diameter Rb of OD 5.2 mm, and the ring C an outer diameter Rc of 5.3 mm. The two rings B and C form a press-fit with the inner circumferential surface of the cylindrical opening 11, wherein the inner circumferential surface is deformed elastically, to create a closed labyrinth.

The pressing depths in press fits of rings B and C thereby amounts to 0.05 mm at ring B, and 0.1 mm at ring C.

As can be seen in Fig. 9, the distances along the longitudinal axis 1 of the centers of the circular contours of the rings A and C are spaced equally from the center of the circular contour of ring B.

When regarding the convex contours of one of rings A, B, and C, it is to be noted that with regard to a symmetry axis 6, which extends through the vertex of the protrusion, the contour is symmetrically shaped to both sides, i.e. in insertion direction 2 and the opposite direction, which is the extraction direction. In other words, by using a circular convex contour for each of rings A, B, C, and/or a circular concave contour to connect the convex contours, the contour seal created to the female connector may easily be moved along the longitudinal axis, specifically may easily be released.

By means of these respective press-fits, the system is sealed in a liquid-tight and air-tight manner, wherein possible adhesion of the liquids is thereby suppressed.

Fig. 10 schematically illustrates an inclination angle of protrusions, according to embodiments of the invention.

In Fig. 10, an upper half of the tubular end section 21 is depicted, wherein the outer diameters of rings A, B, C are exaggerated and not to scale, in order to show the inclination angle defined by rings A, B, C.

As can be seen in Fig. 10, the outer diameters of rings A, B, C define a conical surface 3, which is angled towards the longitudinal axis 1 through the center of the male connector and the female connector. The angle 4 between the conical surface 3 and the longitudinal axis 1 may be characteristic for a pressing ratio of the connector system 100.

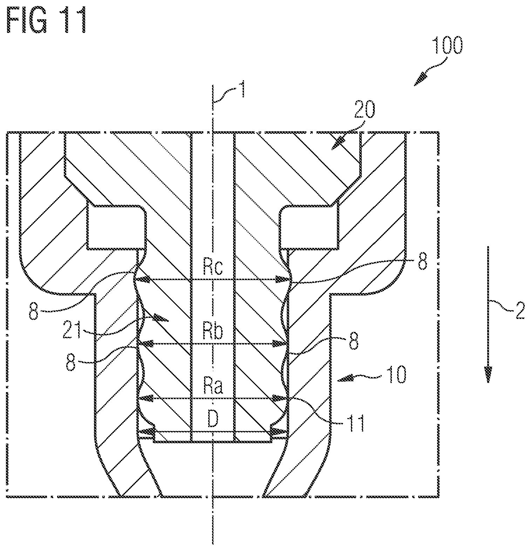

Fig. 11 schematically illustrates the tubular end section 21 of Fig. 9 inserted into a cylindrical opening 11 of a female connector 10.

As can be seen in Fig. 11, in the connector system 100, a press-fit seal 8 is formed between female and male connectors 10, 20 at the outer diameter Rc of protrusion, or ring, C. A further press-fit seal 8 is formed at outer diameter Rb of ring B, wherein ring A does not form a press fit and contacts the inner surface of the cylindrical opening 11 having a diameter of D.

From the above said, the following general conclusions may be drawn.

In various examples, the fluid passages of the female and male connectors are cylindrical with the same diameter, wherein the inner passages are merged at the end surfaces of the male and female connectors, which may be perpendicular end surfaces with respect to the longitudinal axis, to form a continuous cylindrical inner passage for the fluid.

In general, by the disclosed techniques a releasable seal may be provided, wherein the connected male and female connectors may be disconnected, in other words removed or disassembled, from each other, and wherein any elastic deformations are removed, wherein the female and male parts are restored into their original dimensions, i.e. no plastic deformation may be applied to the connectors while providing a liquid-tight, i.e. fluid-tight, seal.

In general, the contact points between the protrusions and the inner surface of the female connector cylindrical opening, may lie on a cone, in other words may define a conical surface, wherein such a cone over the contact points may define a pressing ratio. A pressing ratio may be adjusted based on the materials used in the connectors, and/or the maximum fluid pressure in the fluid system, and/or the mechanical stress on the coupling, or connector, system.

The three protrusions may be referred to a contour of the male connector, specifically the outer surface of the tubular end section. Respectively, the seal provided by the protrusions may be referred to as a contour seal, specifically a releasable contour seal.

A distance between the centers of the protrusions, along the longitudinal axis, may be defined using the outer diameters of the protrusions, and/or the radial distances of the protrusions, and/or the contours of the protrusions. In particular, when circular contours are used for the protrusions, the distance between the centers of the circular contours in longitudinal direction may be in the range of a factor 1.5 to 2 of the radius of the circular contours.

An arrangement along the longitudinal axis may be refer to as an alignment with the longitudinal axis, such that an axis of a respective part lies on the longitudinal axis.

In various examples, the three contours may be circular contours with the same radius, wherein the center of the circles are shifted in the radial direction by steps of 0.1 of the radius.

In various examples, the contours of the protrusions have a round shape, and/or a circular, and/or a symmetrical shape with regard to both the insertion and extraction direction, has the advantage that the connection and thereby, the liquid-tight seal may be easily disconnected. In this regard, the male connector may provide a nozzle to be inserted into e.g. a tube or a pipe, wherein the insertion force is equal to the extraction force, in contrast to known coupling nozzles, which have hook-shaped, or arrow-shaped protrusions.

In various examples, the parts of the contours, which are in contact during a press-fit have a convex shape as explained above, wherein the rest of the contour may be concave, specifically the protrusions may be formed according to injection moulding design rules, in particular such that the connectors may be suitable for forced demoulding, wherein plastic parts of a tough, elastic material are deformed in the elastic range during ejection. The contours may be adapted based on the specific materials used for the female and male connectors, such that according to the present concept an elastic deformation is effected during sealing, i.e. the press-fit, when the connectors are assembled.

In various examples, both the female and male connectors may be made of a hard, or solid, or rigid material, in particular a plastic material, such as Polyetheretherketon (PEEK), in contrast to elastomers and metallic materials.

In various examples, the first protrusion may be in loose or interrupted contact during insertion for centering the male connector in the female connector, or may only be in sliding contact, not deforming the cylindrical opening, with in the inner surface of the cylindrical opening, whereby the first protrusion will not contribute to the liquid-tight sealing between the connector parts, but may be used for aligning the connector parts, specifically along the longitudinal axis, before inserting the intermediate and rear protrusion into the cylindrical opening.

It is to be understood, that the material of the female connector is softer than the material of the male connector.

In general, the female connector may form the cylindrical opening as a cylindrical cavity, for example, having an inner diameter, which is relevant for the dimensions of the male protrusions and tubular end section. Further, the cylindrical opening may be formed in a receiving section of the female connector having an outer surface extending around the cylindrical opening, in other words, around the cylindrical opening there may be a wall, with a thickness with respect to the inner and outer surface in radial direction, wherein, it is to be understood that only the inner the circumferential surface is elastically deformed by the press-fit, wherein an outer surface of the female connector, specifically outer surface directly over the cylindrical opening in the radial direction, has no elastic deformation. In other words, the in the contour of the female connector may be deformed, while the outer contour of the female connector is not deformed. For example, a wall thickness around the inner cylindrical opening along the complete surface, which may form an interference fit with the annular protrusions, have a thickness, allows an elastic deformation of the inner circumferential surface, but no deformation of the outer contour around the cylindrical opening. In this regard, the difference between the outer diameter of a protrusion and the inner diameter of the cylindrical diameter may define a pressing depth into the cylindrical opening, which may not exceed a factor 0.4, or 0.3, or 0.2, 0.1, or 0.05 multiplied by the wall thickness around the cylindrical opening. In various examples, the female connector may not be an elastic hose, or any hose made from an elastomeric material, for example rubber. In various examples, the interference fit may be formed in the elastomeric deformation range of a thermoplastic material used for the female and/or the male connector. Specifically, the elastomeric deformation in the female connector may have a young modulus which is smaller than the young modulus of the male connector, thus leading to an elastic deformation of the cylindrical opening.

In various examples, a baffle coupling, a claw coupling, or a bayonet coupling may be used to hold the female and male connectors together in an assembled state.

The distances between the center points of the contours of the first, intermediate, and rear protrusions, and the radial distances of the center points may be chosen, such that a conical surface with a predefined, material- and use-specific inclination angle may be achieved.

In various examples, in longitudinal direction, there may be no abutment surfaces between the male connector and the female connector necessary.

The connector system may also be referred to as fitting system in a fluid system. The male connector may specifically be connected with a piercing needle and may thus provide a releasable contour seal on the piercing needle.

Summarizing, a connector system is provided, which generates a liquid-tight seal by two press-fits between annular rings of a male connector and a cylindrical opening wall of a female connector. A second press-fit has a stronger pressing dimension into the wall of the cylindrical opening, than a first press fit. A first annular ring may be used for centering of the male connector in the cylindrical opening. In preferred examples, each annular ring in a press fit has the same contour in both insertion and extraction direction, specifically a circular contour with different radial heights. The centering annular ring may have the same diameter as the cylindrical opening for precise centering and mechanical stability.

Thereby, as plastics injection-moulded parts, the requirements may be easily met. The seal may be both liquid-tight and airtight. The seal works in all common diameters in metric and inch as long as the press ratio is maintained. The seal may work independently of the material, as the outer material is only minimally deformed in the elastic range. The shape of the seal may be independent of the liquids used and the length of the exposure time. The seal may not require a stop in the insertion direction, as for example when pressing an O-ring. No tools or a defined torque are required for pre-assembly and assembly of the fitting system.

## Claims

1. , A connector system (100) providing a releasable, liquid-tight seal for a fluid system, the connector system (100) comprising:
a female connector (10), having a cylindrical opening (11) along a longitudinal axis with an inner circumferential surface; and
a male connector (20), having a tubular end section (21) along the longitudinal axis, which is to be inserted in an insertion direction into the cylindrical opening (11), wherein inside the tubular end section (21) a fluid passage (23) is formed along the longitudinal axis, and wherein on an outer circumferential surface (24) of the tubular end section (21), annular protrusions (B, C) are integrally formed, the annular protrusions (B, C) comprising:
an intermediate protrusion (B) having an outer diameter (Rb), which is bigger than an inner diameter (D) of the cylindrical opening (11), and
a rear protrusion (C) located with respect to the insertion direction behind the intermediate protrusion (B) having an outer diameter (Rc), which is bigger than the outer diameter (Rb) of the intermediate protrusion (B);
wherein, when the tubular end section (21) with the annular protrusions (B, C) is inserted into the cylindrical opening (11), the intermediate protrusion (B) and the rear protrusion (C) each form a press-fit with the inner circumferential surface of the cylindrical opening (11), whereby a liquid-tight seal between the male connector (20) and the female connector (10) is generated,
wherein the annular protrusions further comprise an annular front protrusion located with respect to the insertion direction in front of the intermediate protrusion, which has an outer diameter, which is the same or smaller as the inner diameter of the cylindrical opening;
wherein a contour of a protrusion (A, B, C) is defined by the shape of its outer surface along the longitudinal axis; and
the region of the contour of a protrusion (A, B, C), which is in contact with the female connector (10) in a press-fit, has the same convex shape in insertion and extraction direction along the longitudinal axis, wherein the male connector is made of a single material, and wherein the female connector (10) is made of a single material, which is more elastic than a material of the male connector (20), wherein no elastomeric component is used in the connection system to generate the liquid-tight seal.

2. . The connector system according to claim 1, wherein:
the outer diameters (Ra, Rb, Rc) of the protrusions (A, B, C) are defined by the outermost points in radial direction on the circumferential outer surfaces of each protrusion (A, B, C); and
the outer diameters (Ra, Rb, Rc) of the protrusions (A, B, C) define a conical surface (3) around the longitudinal axis and wherein preferably:
the conical surface (3) has an inclination angle relative to the longitudinal axis in the range of 0.5 to 1 degree.

3. . The connector system according to claim 1, wherein:
the protrusions (A, B, C) are rotationally symmetrical with regard to the longitudinal axis; and
the protrusions (A, B, C) have the same contour arranged in different radial distances from the longitudinal axis.

4. . The connector system according to claim 1, wherein: the protrusions (A, B, C) have a circular contour with the same radius.

5. . The connector system according to claim 1, wherein:
the female connector (10) further forms a fluid passage (13) along the longitudinal axis adjacent to the cylindrical opening (11), wherein the fluid passages (13/23) of the female and male connectors (10/20) have the same cross section; and
an end surface of the tubular end section (21) of the male connector (20) abuts an end surface of the fluid passage (13) of the female connector (10) in longitudinal direction, such that the fluid passages (13/23) of the female and male connectors (10/20), are seamlessly merged.

6. . The connector system according to claim 1, wherein:
one of the female or the male connector (10/20) further comprises a union thread on its outer circumferential surface (24);
the other one of the female or the male connector (10/20), comprises an annular union protrusion; and
the connector system (100) further comprises a union nut mechanically engaging the union protrusion and the union thread, wherein the female and the male connectors (10/20), are held in an inserted state.

7. . The connector system according to claim 1, wherein the male and the female connectors (10/20) are monolithic plastic injection-moulded parts.

8. **.** The connector system according to claim 1, wherein the female and male connectors (10/20) are configured to be connected in a linear movement, without rotational movement, along the longitudinal axis to generate a liquid-tight seal.

9. **.** The connector system according to claim 1, wherein between the intermediate protrusion (B), the rear protrusion (C), and the inner circumferential surface of the cylindrical opening (11), a closed labyrinth is generated.

10. . An automatic analyzer comprising:
a connector system (100) according to claim 1 providing a releasable, liquid-tight seal for a fluid system.

11. . The automatic analyzer as claimed in claim 10, wherein the automatic analyzer further comprises at least one receiving position for a liquid vessel.

12. . The automatic analyzer as claimed in claim 10, further comprising at least one robotically displaceable transfer arm.

## Patentansprüche

1. Verbindersystem (100), das eine lösbare, flüssigkeitsdichte Abdichtung für ein Fluidsystem bereitstellt, wobei das Verbindersystem (100) Folgendes umfasst:
einen Buchsenverbinder (10), der eine zylindrische Öffnung (11) entlang einer Längsachse mit einer Innenumfangsfläche aufweist, und
einen Steckverbinder (20), der einen rohrförmigen Endabschnitt (21) entlang der Längsachse aufweist, der in einer Einführrichtung in die zylindrische Öffnung (11) einzuführen ist, wobei innerhalb des rohrförmigen Endabschnitts (21) ein Fluiddurchgang (23) entlang der Längsachse ausgebildet ist und wobei an einer Außenumfangsfläche (24) des rohrförmigen Endabschnitts (21) ringförmige Vorsprünge (B, C) integral ausgebildet sind, wobei die ringförmigen Vorsprünge (B, C) Folgendes umfassen:
einen Zwischenvorsprung (B) mit einem Außendurchmesser (Rb), der größer als ein Innendurchmesser (D) der zylindrischen Öffnung (11) ist, und
einen hinteren Vorsprung (C), der sich in Bezug auf die Einführrichtung hinter dem Zwischenvorsprung (B) befindet und einen Außendurchmesser (Rc) aufweist, der größer als der Außendurchmesser (Rb) des Zwischenvorsprungs (B) ist,
wobei der Zwischenvorsprung (B) und der hintere Vorsprung (C) jeweils eine Presspassung mit der Innenumfangsfläche der zylindrischen Öffnung (11) bilden, wenn der rohrförmige Endabschnitt (21) mit den ringförmigen Vorsprüngen (B, C) in die zylindrische Öffnung (11) eingeführt wird, wodurch eine flüssigkeitsdichte Abdichtung zwischen dem Steckverbinder (20) und dem Buchsenverbinder (10) erzeugt wird,
wobei die ringförmigen Vorsprünge ferner einen ringförmigen vorderen Vorsprung umfassen, der sich in Bezug auf die Einführrichtung vor dem Zwischenvorsprung befindet und einen Außendurchmesser aufweist, der gleich oder kleiner als der Innendurchmesser der zylindrischen Öffnung ist,
wobei eine Kontur eines Vorsprungs (A, B, C) durch die Form seiner Außenfläche entlang der Längsachse definiert ist und
der Bereich der Kontur eines Vorsprungs (A, B, C), der mit dem Buchsenverbinder (10) in einer Presspassung in Kontakt steht, in Einführ- und Ausziehrichtung entlang der Längsachse die gleiche konvexe Form aufweist, wobei der Steckverbinder aus einem einzigen Material hergestellt ist und wobei der Buchsenverbinder (10) aus einem einzigen Material hergestellt ist, das elastischer als ein Material des Steckverbinders (20) ist, wobei keine elastomere Komponente in dem Verbindungssystem verwendet wird, um die flüssigkeitsdichte Abdichtung herzustellen.

2. Verbindersystem nach Anspruch 1, wobei:
die Außendurchmesser (Ra, Rb, Rc) der Vorsprünge (A, B, C) durch die in radialer Richtung äußersten Punkte auf den Umfangsaußenflächen jedes Vorsprungs (A, B, C) definiert sind und
die Außendurchmesser (Ra, Rb, Rc) der Vorsprünge (A, B, C) eine konische Fläche (3) um die Längsachse herum definieren und wobei vorzugsweise:
die konische Fläche (3) einen Neigungswinkel im Bereich von 0,5 bis 1 Grad bezüglich der Längsachse aufweist.

3. Verbindersystem nach Anspruch 1, wobei:
die Vorsprünge (A, B, C) bezüglich der Längsachse rotationssymmetrisch sind und
die in unterschiedlichen radialen Abständen von der Längsachse angeordneten Vorsprünge (A, B, C) die gleiche Kontur aufweisen.

4. Verbindersystem nach Anspruch 1, wobei:
die Vorsprünge (A, B, C) eine kreisförmige Kontur mit dem gleichen Radius haben.

5. Verbindersystem nach Anspruch 1, wobei:
der Buchsenverbinder (10) ferner entlang der Längsachse und der zylindrischen Öffnung (11) benachbart einen Fluiddurchgang (13) bildet, wobei die Fluiddurchgänge (13/23) des Buchsen- und des Steckverbinders (10/20) denselben Querschnitt haben und
eine Endfläche des rohrförmigen Endabschnitts (21) des Steckverbinders (20) in Längsrichtung an einer Endfläche des Fluiddurchgangs (13) des Buchsenverbinders (10) anliegt, so dass die Fluiddurchgänge (13/23) des Buchsen- und des Steckverbinders (10/20) nahtlos ineinander übergehen.

6. Verbindersystem nach Anspruch 1, wobei:
der Buchsen- oder der Steckverbinder (10/20) ferner ein Verschraubungsgewinde an seiner Außenumfangsfläche (24) umfasst,
der jeweils andere des Buchsen- oder Steckverbinders (10/20) einen ringförmigen Verschraubungsvorsprung umfasst und
das Verbindersystem (100) ferner eine Überwurfmutter umfasst, die den Verschraubungsvorsprung und das Verschraubungsgewinde mechanisch in Eingriff nimmt, wobei der Buchsen- und der Steckverbinder (10/20) in einem eingeführten Zustand gehalten werden.

7. Verbindersystem nach Anspruch 1, wobei der Steck- und der Buchsenverbinder (10/20) monolithische Kunststoffspritzgussteile sind.

8. Verbindersystem nach Anspruch 1, wobei der Buchsen- und der Steckverbinder (10/20) dazu ausgestaltet sind, in einer linearen Bewegung ohne Drehbewegung entlang der Längsachse verbunden zu werden, um eine flüssigkeitsdichte Abdichtung herzustellen.

9. Verbindersystem nach Anspruch 1, wobei zwischen dem Zwischenvorsprung (B), dem hinteren Vorsprung (C) und der Innenumfangsfläche der zylindrischen Öffnung (11) ein geschlossenes Labyrinth erzeugt wird.

10. Automatisches Analysegerät, umfassend:
ein Verbindersystem (100) nach Anspruch 1, das eine lösbare, flüssigkeitsdichte Abdichtung für ein Fluidsystem bereitstellt.

11. Automatisches Analysegerät nach Anspruch 10,
wobei das automatische Analysegerät ferner mindestens eine Aufnahmeposition für ein Flüssigkeitsgefäß umfasst.

12. Automatisches Analysegerät nach Anspruch 10, ferner umfassend mindestens einen robotisch verschiebbaren Übertragungsarm.

## Revendications

1. Système de connecteur (100) fournissant un joint libérable et étanche aux liquides pour un système de fluide, le système de connecteur (100) comprenant :
un connecteur femelle (10), ayant une ouverture cylindrique (11) le long d'un axe longitudinal avec une surface circonférentielle intérieure ; et
un connecteur mâle (20), ayant une section d'extrémité tubulaire (21) le long de l'axe longitudinal, qui doit être inséré dans une direction d'insertion dans l'ouverture cylindrique (11), dans lequel un passage de fluide (23) est formé à l'intérieur de la section d'extrémité tubulaire (21) le long de l'axe longitudinal, et dans lequel sur une surface circonférentielle extérieure (24) de la section d'extrémité tubulaire (21), des saillies annulaires (B, C) sont formées intégralement, les saillies annulaires (B, C) comprenant :
une saillie intermédiaire (B) dont le diamètre extérieur (Rb) est supérieur au diamètre intérieur (D) de l'ouverture cylindrique (11), et
une saillie arrière (C) située par rapport à la direction d'insertion à l'arrière de la saillie intermédiaire (B) dont le diamètre extérieur (Rc) est supérieur au diamètre extérieur (Rb) de la saillie intermédiaire (B) ;
dans lequel, lorsque la section d'extrémité tubulaire (21) avec les saillies annulaires (B, C) est insérée dans l'ouverture cylindrique (11), la saillie intermédiaire (B) et la saillie arrière (C) forment chacune un ajustement serré avec la surface circonférentielle intérieure de l'ouverture cylindrique (11), ce qui permet de créer un joint étanche aux liquides entre le connecteur mâle (20) et le connecteur femelle (10),
dans lequel les saillies annulaires comportent en outre une saillie avant annulaire située par rapport à la direction d'insertion devant la saillie intermédiaire, qui a un diamètre extérieur identique ou inférieur au diamètre intérieur de l'ouverture cylindrique ;
dans lequel un contour d'une saillie (A, B, C) est défini par la forme de sa surface extérieure le long de l'axe longitudinal ; et
la région du contour d'une saillie (A, B, C), qui est en contact avec le connecteur femelle (10) dans un ajustement serré, a la même forme convexe dans la direction de l'insertion et de l'extraction le long de l'axe longitudinal, dans lequel le connecteur mâle est constitué d'un seul matériau, et dans lequel le connecteur femelle (10) est constitué d'un seul matériau, qui est plus élastique qu'un matériau du connecteur mâle (20), dans lequel aucun élément élastomère n'est utilisé dans le système de connexion pour générer le joint étanche aux liquides.

2. Système de connecteur selon la revendication 1, dans lequel :
les diamètres extérieurs (Ra, Rb, Rc) des saillies (A, B, C) sont définis par les points les plus extérieurs dans la direction radiale sur les surfaces extérieures circonférentielles de chaque saillie (A, B, C) ; et
les diamètres extérieurs (Ra, Rb, Rc) des saillies (A, B, C) définissent une surface conique (3) autour de l'axe longitudinal et où, de préférence :
la surface conique (3) présente un angle d'inclinaison par rapport à l'axe longitudinal compris entre 0,5 et 1 degré.

3. Système de connecteur selon la revendication 1, dans lequel :
les saillies (A, B, C) sont symétriques en rotation par rapport à l'axe longitudinal ; et
les saillies (A, B, C) ont le même contour et sont disposées à des distances radiales différentes de l'axe longitudinal.

4. Système de connecteur selon la revendication 1, dans lequel :
les saillies (A, B, C) ont un contour circulaire avec le même rayon.

5. Système de connecteur selon la revendication 1, dans lequel :
le connecteur femelle (10) forme en outre un passage de fluide (13) le long de l'axe longitudinal adjacent à l'ouverture cylindrique (11), dans lequel les passages de fluide (13/23) des connecteurs femelle et mâle (10/20) ont la même section transversale ; et
une surface d'extrémité de la section d'extrémité tubulaire (21) du connecteur mâle (20) est en contact avec une surface d'extrémité du passage de fluide (13) du connecteur femelle (10) dans la direction longitudinale, de sorte que les passages de fluide (13/23) des connecteurs femelle et mâle (10/20) sont fusionnés sans soudure.

6. Système de connecteur selon la revendication 1, dans lequel :
un du connecteur femelle ou mâle (10/20) comporte en outre un filetage d'accouplement sur sa surface circonférentielle extérieure (24) ;
l'autre du connecteur femelle ou mâle (10/20), comporte une saillie d'accouplement annulaire ; et
le système de connecteur (100) comprend en outre un écrou d'accouplement qui engage mécaniquement la saillie d'accouplement et le filetage d'accouplement, dans lequel les connecteurs femelle et mâle (10/20) sont maintenus dans un état inséré.

7. Système de connecteur selon la revendication 1, dans lequel les connecteurs mâle et femelle (10/20) sont des pièces monolithiques en plastique moulées par injection.

8. Système de connecteur selon la revendication 1, dans lequel les connecteurs femelle et mâle (10120) sont configurés pour être connectés dans un mouvement linéaire, sans mouvement de rotation, le long de l'axe longitudinal pour générer un joint étanche aux liquides.

9. Système de connecteur selon la revendication 1, dans lequel un labyrinthe fermé est généré entre la saillie intermédiaire (B), la saillie arrière (C) et la surface circonférentielle intérieure de l'ouverture cylindrique (11).

10. Analyseur automatique comprenant:
un système de connecteur (100) selon la revendication 1 fournissant un joint libérable et étanche aux liquides pour un système de fluide.

11. Analyseur automatique selon la revendication 10, dans lequel l'analyseur automatique comprend en outre au moins une position de réception pour un récipient de liquide.

12. Analyseur automatique selon la revendication 10, comprenant en outre au moins un bras de transfert déplaçable robotiquement.
